# FASCICULE DE BREVET EUROPEEN

(11) **EP 0 633 872 B1**
(45) Date de publication et mention de la délivrance du brevet: **11.09.1996**
(21) Numéro de dépôt: 93907908.3
(22) Date de dépôt: 30.03.1993
(51) Int. Cl.: C07C 15/073, C07C 2/66

(54) **PROCEDE DE FABRICATION D'ETHYLBENZENE**
Verfahren zur Herstellung von Ethylbenzol
METHOD FOR MANUFACTURING ETHYLBENZENE

(30) Priorité: 01.04.1992 FR 9203955
(43) Date de publication de la demande: 18.01.1995
(73) Titulaire: ELF ATOCHEM S.A., F-92800 Puteaux (FR)
(72) Inventeur: DEMAY, Claude, F-92800 Puteaux (FR)
(74) Mandataire: Kaplan, Jean-Pierre
(86) Numéro de dépôt international: FR9300316
(87) Numéro de publication internationale: WO9320029

(56) Documents cités:
- EP-A- 0 467 007
- GB-A- 1 013 268
- US-A- 3 200 164
- US-A- 3 205 277
- US-A- 3 848 012
- US-A- 4 459 426
- CHEMICAL ABSTRACTS, vol. 79, 1973, Columbus, Ohio, US; abstract no. 125971e, Y.I. KOZOREZOV 'Benzene alkylation by ethylene diluted in a catalyst composed of boron fluoride on aluminium oxide'
- CHEMICAL ABSTRACTS, vol. 90, 1979, Columbus, Ohio, US; abstract no. 86974p, S. HATTORI 'Ethylbenzene'

## Description

La présente invention se rapporte au domaine technique du raffinage pétrolier et de la pétrochimie et plus précisément concerne un procédé de fabrication d'éthylbenzène à partir d'une composition comportant du benzène et d'une autre composition comportant de l'éthylène. L'éthylbenzène trouve son utilité essentiellement comme matière première dans la fabrication du styrène.

Les procédés connus de fabrication de l'éthylbenzène utilisent la réaction du type Friedel et Crafts, d'alkylation du benzène par l'éthylène.

Les catalyseurs de cette réaction sont des acides de Brönsted ou de Lewis, choisis notamment parmi le chlorure d'aluminium, le trifluorure de bore déposé sur alumine, ou encore des zéolithes utilisées en phase liquide ou gazeuse.

Une des difficultés rencontrées dans la mise en oeuvre de cette réaction résulte du fait que l'éthylbenzène formé est plus réactif que le benzène vis-à-vis de l'éthylène, ce qui conduit à des diéthylbenzènes qui, eux-mêmes, plus réactifs que l'éthylbenzène ont tendance à former des triéthylbenzènes.

Cette tendance vers une hexa substitution du benzène est cependant limitée par des effets stériques et par le fait que les composés aromatiques ainsi produits de plus en plus riches en électrons finissent par former des complexes très stables avec les catalyseurs électroaccepteurs utilisés dans cette réaction d'alkylation.

Actuellement, pour limiter ces réactions de polyalkylation, on utilise un fort excès de benzène par rapport à l'éthylène à l'entrée des réacteurs d'alkylation.

Ainsi, le rapport molaire benzène/éthylène est en général compris entre 2 et 2,5 pour les procédés utilisant le chlorure d'aluminium et peut même atteindre une valeur comprise entre 8 et 16 pour les procédés utilisant les zéolithes en phase gazeuse.

Malgré l'utilisation d'un excès de benzène par rapport à l'éthylène pour minimiser la formation de polyéthylbenzènes, celle-ci ne peut être totalement évitée. Ainsi, une composition typique d'un alkylat obtenu en sortie de réacteur est donnée dans l'ouvrage "Procédés de pétrochimie - Edition Technip - Tome I, p. 400, 1985 :
les pourcentages sont donnés en poids par rapport au poids total :

| | |
|---|---|
| Benzène | 38 - 40 % |
| Ethylbenzène | 41 - 43 % |
| Diéthylbenzènes | 12 - 14 % |
| Triéthylbenzènes | 2 - 3 % |
| Polyéthylbenzènes supérieurs ayant un nombre de groupes éthyle supérieur à 3 et divers | 3 - 4 % |

Dans tous les procédés connus, les polyéthylbenzènes, c'est-à-dire les composés phényle substitués par au moins deux groupes éthyle sont isolés avant d'être soumis à une réaction de transalkylation par un excès de benzène selon: 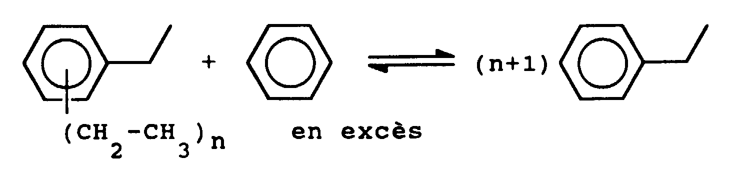 Cette réaction, catalysée par les mêmes catalyseurs que la réaction d'alkylation ci-dessus, peut être mise en oeuvre dans un réacteur séparé de celui de l'alkylation, ou bien dans le réacteur d'alkylation lui-même qui fonctionne avec un excès de benzène pour favoriser au mieux la formation prépondérante d'éthylbenzène.

Une description détaillée de ces techniques connues de fabrication d'éthylbenzène figure dans l'ouvrage "Procédés de pétrochimie" mentionné précédemment ou encore dans "Encyclopedia of Chemical Technology - 3ème édition-John Wiley and Sons - Vol. 21, p. 772 et suivantes".

Dans tous les procédés connus évoqués ci-dessus, le benzène et l'éthylène utilisés dans la réaction d'alkylation sont chacun des produits d'une pureté technique suffisante pour conduire à de l'éthylbenzène technique qui peut être ensuite purifié au degré voulu par une distillation-rectification.

Par ailleurs, les technologies du raffinage pétrolier et des principaux produits qui en résultent ont été décrites dans "Encyclopedia of Chemical Technology-3ème édition - vol. 17, p. 183 et suivantes".

C'est ainsi que certaines fractions lourdes obtenues par distillation du pétrole sont craquées catalytiquement dans le craquage catalytique fluide (F.C.C.) pour donner des produits plus légers qui sont valorisés comme essences à haut indice d'octane. Ce craquage donne également une certaine proportion de gaz dit "gaz de F.C.C." dont une fraction légère comporte des hydrocarbures saturés ayant un nombre de carbones inférieur ou égal à 2 et de l'éthylène. Cet éthylène est présent à une teneur en poids en général inférieure à 30% dans cette fraction légère. La récupération de cet éthylène par une liquéfaction suivie d'une distillation n'est pas économiquement rentable et l'utilisation de cet éthylène dilué essentiellement par du méthane et de l'éthane a déjà été décrite, notamment dans les brevets anglais GB-908505, GB-1 013 268, américains US-A-3 131 230, US-A-3 200 164, US-A-3 205 277.

Dans les procédés d'alkylation de ces brevets, le rapport benzène/éthylène est supérieur à 2 et d'une manière générale au rapport utilisé dans les procédés connus, mettant en oeuvre de l'éthylène pratiquement pur, afin de pouvoir épuiser l'éthylène présent dans ladite fraction légère. (voir également : Process economics program, Standford Research Institute - Report n° 33 - Oct. 1986, p. 37).

L'alkylat obtenu est fortement prépondérant en benzène et comporte une faible teneur en éthylbenzène et polyéthylbenzènes, ce qui entraîne un coût important pour la séparation de l'éthylbenzène de ce mélange et un tel procédé de valorisation de l'éthylène ne semble pas actuellement faire l'objet d'une exploitation industrielle. Ainsi, la fraction légère des gaz de F.F.C. n'est pas valorisée pour son contenu en éthylène.

Le benzène utilisé dans les procédés ci-dessus provient en général de coupes issues d'un vapocraqueur d'une unité pétrochimique, ou encore de coupes issues du reformeur d'une raffinerie de pétrole brut.

Les fractions de tête d'un reformeur peuvent contenir une proportion importante de benzène et sont donc la source d'une part importante de la production mondiale de benzène.

Cependant, lorsque le reformeur est sous-dimensionné par rapport à l'ensemble de la raffinerie ou encore lorsqu'il est alimenté par des fractions trop lourdes pour conduire à des quantités importantes de benzène, ce dernier, pour des considérations économiques, est alors laissé dans les essences. Cependant, compte tenu de la toxicité reconnue du benzène, sa présence dans les essences pose des problèmes vis-à-vis des réglementations existantes ou futures.

Le but principal de la présente invention est de mieux valoriser sur le plan économique l'éthylène dilué, notamment en provenance des gaz du F.F.C.

Un autre but de la présente invention est d'éliminer le benzène des réformats légers, tout en produisant de l'éthylbenzène qui peut être valorisé par déshydrogénation en styrène.

Ces réformats légers, après élimination du benzène, peuvent conduire par distillation-rectification à des essences sans benzène.

Un autre but de la présente invention est d'éliminer le benzène des réformats légers tout en isomérisant la partie majoritaire non benzénique du réformat en produits à indice d'octane amélioré.

Le but principal est atteint selon la présente invention par un procédé de fabrication d'éthylbenzène à partir de benzène pur ou de benzène dilué et d'une composition (B) comportant de l'éthylène, caractérisé en ce qu'il consiste à:
a) faire réagir le benzène pur ou le benzène dilué avec la composition (B) dans une réaction d'alkylation du benzène par l'éthylène dans laquelle le rapport molaire benzène/éthylène est compris entre 0,3 et 1, de façon à obtenir un alkylat pratiquement sans benzène résiduel et comportant du mono-, du di- et du tri- éthylbenzène;
b) soumettre cet alkylat à au moins une distillation-rectification pour obtenir un distillat comportant du di- et du tri- éthylbenzène ;
c) transalkyler ce distillat par du benzène pour obtenir un transalkylat enrichi en monoéthylbenzène formé à partir du di- et du tri- éthylbenzène dudit distillat ;
d) soumettre ce transalkylat à au moins une distillation-rectification pour obtenir de l'éthylbenzène.

Le rapport molaire benzène/éthylène est compris entre 0,3 et 1, de façon à éliminer pratiquement tout le benzène mis en réaction tout en ayant une cinétique suffisamment rapide de la réaction d'alkylation du benzène par l'éthylène.

Avantageusement, la réaction d'alkylation est industriellement praticable à l'aide de benzène comportant également des hydrocarbures saturés diluant le benzène présent.

Avantageusement, le benzène dilué dans des hydrocarbures saturés est un réformat léger en provenance d'une raffinerie de pétrole brut. La possibilité d'utiliser ces réformats légers généralement très abondants et d'y supprimer leur teneur en benzène permet d'accéder à des essences répondant le mieux aux réglementations.

Avantageusement, la composition (B) comporte de l'éthylène dilué dans des hydrocarbures saturés, à l'exclusion d'hydrocarbures insaturés différents de l'éthylène. Ainsi, l'absence, par exemple, de propylène simplifie la composition de l'alkylat obtenu et permet une séparation aisée de ses constituants notamment par une ou plusieurs distillations-rectifications.

De préférence, la composition (B) est une fraction de distillation d'un gaz de "Fluid Catalytic Cracking" comportant des hydrocarbures ayant un nombre de carbones inférieur ou égal à 2.

Lorsque le benzène dilué est une fraction légère de réformat disponible dans une raffinerie, on voit alors tout l'intérêt du procédé selon l'invention qui permet alors de traiter la majeure partie de l'éthylène contenu dans les gaz de F.F.C. de la raffinerie avec la totalité du benzène disponible dans le réformat léger.

D'une manière préférée, la ou les distillation(s)-rectification(s) de l'étape b) est(sont) réglée(s) de manière que ledit distillat de l'étape b) ne comporte plus de mono-éthylbenzène,ce qui est obtenu par au moins une distillation-rectification pour obtenir une fraction de tête comportant du monoéthylbenzène et une fraction de queue comportant du di- et du tri-éthylbenzène et des lourds.

D'une manière préférée, cette fraction de tête comportant du mono-éthylbenzène est recyclée dans l'étape a) pour obtenir l'alkylation de ce mono-éthylbenzène en di- et tri-éthylbenzène. De cette manière, l'alkylat sortant du réacteur d'alkylation est encore plus enrichi en di- et tri-éthylbenzène que l'alkylat obtenu sans recyclage et le benzène contenu dans le benzène dilué se combine à un plus grand nombre de molécules d'éthylène en étant un meilleur vecteur du groupe éthyle dans la transalkylation de l'étape c).

Avantageusement, le benzène dilué étant un réformat léger, à l'étape b) on récupère, par une autre distillation-rectification, une fraction essence formée d'hydrocarbures saturés et essentiellement sans benzène.

La transalkylation de l'étape c) du procédé selon la présente invention est pratiquée selon des procédés connus, le courant de benzène utilisé étant alors ajusté pour respecter les rapports benzène/groupes éthyle habituellement utilisés. Cette transalkylation peut être mise en oeuvre sur le site même où est pratiquée l'alkylation de l'étape a) ou sur un autre site disposant de quantités suffisantes de benzène. Dans ce dernier cas, les polyéthylbenzènes à base de di- et de tri-éthylbenzène peuvent être introduits dans une unité d'alkylation classique au niveau du réacteur d'alkylation ou de transalkylation.

En plus de la description qui précède, l'invention sera mieux comprise à l'aide des exemples qui vont suivre et parmi lesquels l'Exemple 5 est décrit en référence à la figure unique annexée représentant un schéma d'un mode de réalisation du procédé.

Ces exemples sont donnés à titre purement illustratif sans vouloir aucunement limiter la portée de l'invention.

### EXEMPLE 1

Dans un autoclave émaillé, résistant à la pression, muni d'un agitateur, on introduit 1 000 g d'une charge de réformat léger déshydraté par passage sur un tamis moléculaire et contenant en pourcentage en poids :
- des paraffines en C-6 (à 6 atomes de carbone) : 72 %
- des cycloparaffines en C-6 (à 6 atomes de carbone): 22 %
- du benzène : 6 %,
on ajoute 1 g de chlorure d'aluminium AlCl₃ et 1,4 g de chlorure d'éthyle, puis l'on ferme l'autoclave.

Après mise en route de l'agitation, on porte la température de l'autoclave à 160°C. Par une vanne, on injecte alors dans l'autoclave un gaz sous pression, équivalent à 180 litres dans les conditions normales de pression et de température, et ayant la composition molaire suivante :
- méthane 51 %
- éthane 33 %
- éthylène 16 %

Compte tenu des groupes éthyle introduits sous la forme de chlorure d'éthyle, le rapport molaire benzène/éthylène est égal à 0,59.

Au bout d'une heure, après refroidissement et détente des gaz dans un piège à carboglace (-78°C), les fractions liquides, récupérées dans l'autoclave et le piège, sont rassemblées, lavées à l'eau, neutralisées par lavage avec une solution 1 N de soude, puis de nouveau lavées à l'eau.

La composition pondérale de la fraction lourde contenant les composés hydrocarbures ayant au moins huit atomes de carbone, déterminée par chromatographie en phase vapeur, est la suivante :
- éthylbenzène 41 %
- diéthylbenzène 43 %
- triéthylbenzène 13 %
- lourds 3 %

Par distillation, après élimination de la fraction de tête constituée d'hydrocarbures ayant 6 atomes de carbone, on récupère 38 g d'éthylbenzène et 52 g d'un mélange de diéthylbenzène et de triéthylbenzène de composition pondérale :
- diéthylbenzène 77 %
- triéthylbenzène 23 %

En principe, les 38 g d'éthylbenzène obtenus pourraient être recyclés jusqu'à disparition dans la même réaction d'alkylation.

Ils représentent ainsi un potentiel d'environ 50 g d'un mélange diéthylbenzène/triéthylbenzène de composition pondérale 77/23.

Ainsi, la mise en oeuvre de la réaction d'alkylation dans les conditions décrites ci-dessus, avec un rapport molaire benzène/éthylène égal à 0,59 et recyclage total de l'éthylbenzène formé permet d'obtenir, théoriquement à partir des 1 000 g de réformat léger, environ 102 g d'un mélange diéthylbenzène/triéthylbenzène de 77/23 en poids.

On introduit les 52 g du mélange de di et de tri-éthylbenzènes 77/23 précédent dans un autoclave contenant 120 g de benzène, 0,17 g de chlorure d'aluminium et 0,24 g de chlorure d'éthyle. Le rapport molaire du benzène/groupes éthyle est égal à 2,3.

L'autoclave est fermé, l'agitation mise en route et la température est élevée à 160°C.

Après 1 heure de réaction à cette température, on refroidit avant d'égaliser la pression dans l'autoclave, et d'ouvrir celui-ci. Le contenu est neutralisé et lavé comme ci-dessus après la réaction d'alkylation. On obtient 172 g d'un mélange contenant en poids :
- benzène 50 %
- éthylbenzène 40 %
- diéthylbenzène 7 %
- Lourds 1 %

Ce mélange est soumis à une distillation-rectification et conduit ainsi à environ 68 g d'éthylbenzène et 11 g de diéthylbenzène.

Ce diéthylbenzène peut être recyclé dans une réaction de transalkylation pour augmenter le rendement en éthylbenzène.

Les calculs montrent que la totalité d'éthylbenzène fabricable à partir des 52 g du mélange mis en oeuvre se monte à environ 86 g.

A partir des 102 g du mélange diéthylbenzène-triéthylbenzène potentiellement accessible à partir des 180 1 de gaz ci-dessus, l'éthylbenzène obtenable se monte à environ 169 g.

Ainsi, 1 000 g du réformat léger peuvent conduire à environ 169 g d'éthylbenzène.

### EXEMPLE 2 - COMPARATIF

A titre de comparaison, on traite 1 000 g du même réformat léger, dans les mêmes conditions, que l'Exemple 1, sauf que l'on utilise un rapport molaire benzène/éthylène égal à 2,5, pour minimiser comme dans les technologies connues, la formation des polyéthylbenzènes. On n'injecte donc dans l'autoclave que 40 1 du gaz de l'Exemple 1.

Après réaction, refroidissement, neutralisation, lavages comme dans l'Exemple 1, on récupère une phase organique dont la composition pondérale des hydrocarbures ayant au moins 8 atomes de carbone est la suivante :
- éthylbenzène 84 %
- diéthylbenzène 15 %
- triéthylbenzène 1 %

Par distillation-rectification on récupère 22,7 g d'éthylbenzène et 4,2 g de polyéthylbenzène. Ces polyéthylbenzènes recyclés dans une réaction de transalkylation représentent un potentiel d'environ 7 g d'éthylbenzène.

La quantité d'éthylbenzène potentiellement accessible par ce procédé en une étape à partir des 1 000 g de réformat léger se monte donc à environ 29 g.

On s'aperçoit en outre que sur les 60 g de benzène contenus dans le réformat léger, seuls 20 g environ ont réagi. On peut envisager le recyclage de ce réformat, mais ceci est difficilement concevable, compte tenu des taux de dilution du benzène très faibles qui iraient en diminuant avec les recyclages, entraînant la nécessité d'énormes capacités réactionnelles.

Même en retenant cette hypothèse,les calculs montrent que ces recyclages jusqu'à extinction du benzène ne conduiraient qu'à un maximum théorique d'environ 55 g d'éthylbenzène. Ainsi, les 1 000 g de réformat n'ont qu'un potentiel théorique d'environ 85 g d'éthylbenzène,lorsqu'ils sont traités selon ce procédé comparatif.

### EXEMPLE 3

Dans un autoclave en acier inoxydable résistant à la pression et muni d'un agitateur, on introduit 500 g d'une charge de réformat de l'Exemple 1 et 175 g d'une mordénite à petits pores désaluminé, de rapport Si/Al = 25.

On porte la température de l'autoclave fermé à 270°C, puis on injecte, par une vanne, dans l'autoclave l'équivalent de 140 N litres du même gaz que l'Exemple 1.

Le rapport molaire benzène/éthylène est ainsi égal à 0,38. Au bout de deux heures de réaction à 270°C, après refroidissement et détente des gaz comme dans l'Exemple 1, la composition pondérale de la fraction des hydrocarbures ayant au moins 8 atomes de carbone est la suivante :
- éthylbenzène 12 %
- diéthylbenzène 43 %
- triéthylbenzène 31 %
- Lourds 14 %

Par distillation, on récupère 5,5 g d'éthylbenzène et 36 g d'un mélange de polyéthylbenzènes ayant la composition pondérale suivante :
- diéthylbenzène 58 %
- triéthylbenzène 42 %

Le recyclage des 5,5 g d'éthylbenzène ci-dessus pourrait conduire à environ 7,6 g de mélange diéthylbenzène/triéthylbenzène 58/42.

Par recyclage total de l'éthylbenzène formé dans cette réaction d'alkylation, les 500 g du réformat léger pourraient conduire à environ 44 g d'un mélange diéthylbenzène/triéthylbenzène 58/42.

### EXEMPLE 4

Dans un autoclave de transalkylation identique à celui de l'Exemple 1, on introduit les 36 g du mélange de polyéthylbenzènes ci-dessus, 110 g de benzène séché sur tamis moléculaire, 0,15 g de chlorure d'aluminium et 0,22 g de chlorure d'éthyle. On opère comme dans l'Exemple 1 et on récupère 136 g d'un mélange contenant en poids :
- benzène 54 %
- éthylbenzène 38 %
- diéthylbenzène 6 %
- Lourds 2 %

Par distillation-rectification,on récupère 51,7g d'éthylbenzène et 8,2 g de diéthylbenzène qui après recyclage dans la même réaction de transalkylation conduirait à environ 13 g d'éthylbenzène.

A partir des 36 g du mélange mis en oeuvre,on peut ainsi obtenir en recyclant les diéthylbenzènes jusqu'à extinction,67,4 g d'éthylbenzène.

A partir des 44 g du mélange potentiellement accessible en traitant 500 g de réformat par le procédé de l'exemple 3,on peut ainsi obtenir 82,4 g d'éthylbenzène,soit environ 165 g rapporté à 1000 g de raffinat.

### EXEMPLE 5

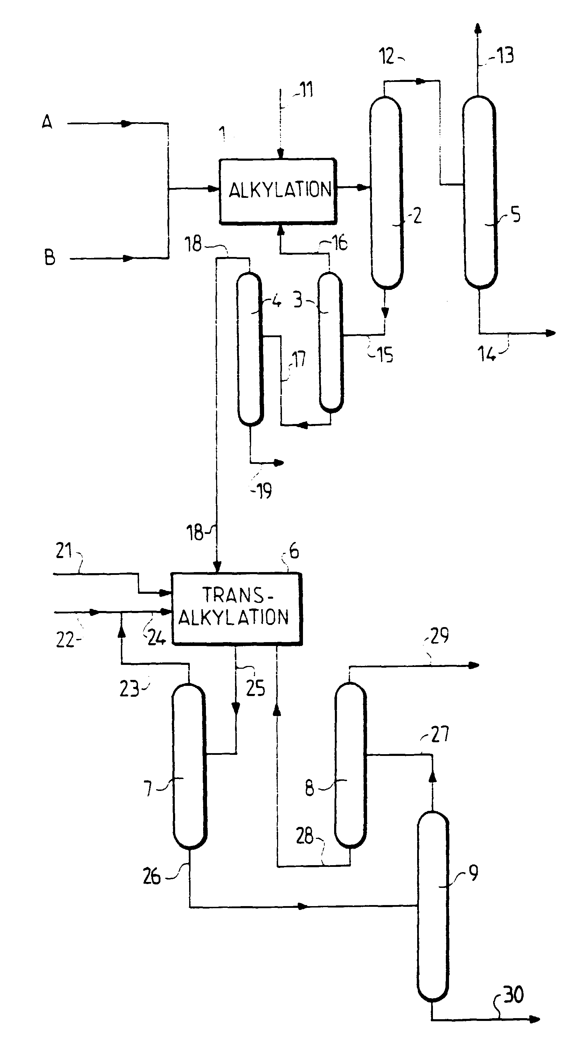

La figure 1 montre un schéma d'un mode de réalisation du procédé dans une installation comportant un réacteur d'alkylation 1, un réacteur de transalkylation 6 et des colonnes à distiller 2 à 5 et 7 à 9.

Les réacteurs et les colonnes sont chacun séparément connus en soi. L'installation fonctionne en continu et les quantités pondérales, volumiques ou molaires, sont indiquées par rapport à 1 000 g de réformat léger.

Ce réformat léger A comporte des paraffines en C-6 et des cycloparaffines en C-6 (ensemble 11 moles), ainsi que du benzène (0,77 mole).

Le gaz de F.F.C. (249 l) B comporte un mélange de méthane et d'éthane (ensemble 9,34 moles) et de l'éthylène (1,78 moles). Cette proportion de réformat A et de gaz B est introduite dans le réacteur d'alkylation 1 avec un catalyseur 11 comprenant de l'AlCl₃ (0,0075 mole) et du chlorure d'éthyle (0,0217 mole). Après un temps de séjour suffisant, le milieu réactionnel obtenu est dirigé vers la colonne 2 qui sépare le milieu en une fraction de tête 12 et une fraction de queue 15. La fraction de tête dirigée sur la colonne 5 est séparée en une fraction gaz 13 comprenant du méthane et de l'éthane (ensemble 9,34 moles) ainsi que de l'éthylène (0,02 mole) et une fraction de queue constituée par des essences 14 (11 moles) sans teneur notable en benzène. La fraction de queue 15 comporte du monoéthylbenzène (0,72mole),du diéthylbenzène (0,59 mole), du triéthylbenzène (0,15mole) et des lourds(0,03 mole). Cette fraction est dirigée sur la colonne 3 qui sépare en tête l'éthylbenzène 16 qui est recyclé dans le réacteur 1, et en queue 17 le di-, le tri- éthylbenzène et les lourds qui sont introduits dans la colonne 4 qui élimine en queue les lourds 19 (0,03 mole).

La colonne 4 sort en tête un distillat 18 (étape b)) comportant du diéthylbenzène (0,59 mole) et du triéthylbenzène
Ce distillat 18 est introduit dans le réacteur de transalkylation 6 avec les catalyseurs 21 comprenant de l'AlCl₃ (0,0013 mole) et du chlorure d'éthyle (0,0037 mole). Du benzène 22 (0,86 mole) est introduit également dans ce réacteur 6. Le transalkylat 25 sortant du réacteur 6 est dirigé vers la colonne 7 qui sépare en tête le benzène 23 qui est recyclé dans le réacteur 6 et en queue un mélange 26 contenant de l'éthylbenzène (1,58 mole), du diéthylbenzène (0,27 mole) et des lourds (0,02 mole). Le mélange 26 est dirigé sur la colonne 9 qui élimine en queue les lourds 30 (0,02 mole) et qui conduit en tête à un mélange 27 d'éthylbenzène (1,58 mole) et de diéthylbenzène (0,27 mole). Le mélange 27 est introduit dans la colonne 8 qui sépare en tête l'éthylbenzène pur ou pratiquement pur 29 (1,58 mole) et en queue le diéthylbenzène 28 (0,27 mole) qui est recyclé dans le réacteur 6.

## Revendications

1. Procédé de fabrication d'éthylbenzène à partir de benzène pur ou de benzène dilué et d'une composition (B) comportant de l'éthylène, caractérisé en ce qu'il consiste à:
a) faire réagir le benzène pur ou le benzène dilué avec la composition (B) dans une réaction d'alkylation du benzène par l'éthylène dans laquelle le rapport molaire benzène/éthylène est compris entre 0,3 et 1, de façon à obtenir un alkylat pratiquement sans benzène résiduel et comportant du mono-, du di- et du tri- éthylbenzène;
b) soumettre cet alkylat à au moins une distillation-rectification pour obtenir un distillat (18) comportant du di- et du tri- éthylbenzène ;
c) transalkyler ce distillat (18) par du benzène (22) pour obtenir un transalkylat (25) enrichi en monoéthylbenzène formé à partir du di- et du tri-éthylbenzène dudit distillat ;
d) soumettre ce transalkylat (25) à au moins une distillation-rectification pour obtenir de l'éthylbenzène (29).

2. Procédé suivant la revendication 1, caractérisé en ce que le benzène dilué comporte des hydrocarbures saturés.

3. Procédé suivant la revendication 1, caractérisé en ce que le benzène dilué est un réformat léger.

4. Procédé suivant l'une des revendications 1 à 3, caractérisé en ce que la composition (B) comporte également des hydrocarbures saturés à l'exclusion d'hydrocarbures insaturés différents de l'éthylène.

5. Procédé suivant la revendication 4, caractérisé en ce que la composition (B) est une fraction de distillation d'un gaz de craquage catalytique fluide comportant des hydrocarbures ayant un nombre de carbones inférieur ou égal à 2.

6. Procédé suivant l'une des revendications 1 à 5, caractérisé en ce que le distillat de l'étape b) comporte également du monoéthylbenzène et en ce qu'on soumet ce distillat à au moins une distillation-rectification pour obtenir une fraction de tête comportant du monoéthylbenzène et une fraction de queue comportant du di- et du tri- éthylbenzène.

7. Procédé suivant la revendication 6, caractérisé en ce que ladite fraction de tête comportant du monoéthylbenzène est recyclée dans l'étape a) pour obtenir l'alkylation de ce monoéthylbenzène en di- et tri-éthylbenzène.

8. Procédé suivant la revendication 3 et l'une des revendications 4 à 7, caractérisé en ce qu'à l'étape b) on récupère, par une autre distillation-rectification, une fraction essence formée d'hydrocarbures saturés et essentiellement sans benzène.

## Patentansprüche

1. Verfahren zur Herstellung von Ethylbenzol aus reinem oder verdünntem Benzol und einer Zusammensetzung (B), die Ethylen enthält,
**gekennzeichnet** durch folgende Schritte:
a) Umsetzung des reinen oder verdünnten Benzols mit der Zusammensetzung (B) in einer Alkylierungsreaktion, bei der Benzol mit Ethylen alkyliert wird und bei der das Molverhältnis Benzol/Ethylen 0,3 bis 1 beträgt, so daß ein Alkylierungsprodukt fast ohne restliches Benzol erhalten wird, das Mono-, Di- und Triethylbenzol enthält,
b) Durchführung mindestens einer Destillation-Rektifikation mit diesem Alkylierungsprodukt unter Erhalt eines Destillats (18), das Di- und Triethylbenzol enthält,
c) Transalkylierung dieses Destillats (18) mit Benzol (22) unter Erhalt eines Transalkylierungsprodukts (25), das mit Monoethylbenzol angereichert ist, das aus dem Di- und Triethylbenzol des Destillats erzeugt wurde,
d) Durchführung mindestens einer Destillation-Rektifikation mit diesem Transalkylierungsprodukt (25) unter Erhalt von Ethylbenzol (29).

2. Verfahren nach Anspruch 1, dadurch gekennzeichnet, daß das verdünnte Benzol gesättigte Kohlenwasserstoffe enthält.

3. Verfahren nach Anspruch 1, dadurch gekennzeichnet, daß das verdünnte Benzol ein Leichtreformat ist.

4. Verfahren nach einem der Ansprüche 1 bis 3, dadurch gekennzeichnet, daß die Zusammensetzung (B) ebenfalls gesättigte Kohlenwasserstoffe enthält, wobei andere ungesättigte Kohlenwasserstoffe als Ethylen ausgeschlossen sind.

5. Verfahren nach Anspruch 4, dadurch gekennzeichnet, daß die Zusammensetzung (B) eine Destillationsfraktion eines durch katalytisches Kracken im Fließbett (FCC) erhaltenen Krackgases ist, die Kohlenwasserstoffe mit höchstens zwei Kohlenstoffatomen enthält.

6. Verfahren nach einem der Ansprüche 1 bis 5, dadurch gekennzeichnet, daß das Destillat aus Schritt b) auch Monoethylbenzol enthält und daß dieses Destillat mindestens einer Destillation-Rektifikation unter Erhalt einer Kopffraktion, die Monoethylbenzol enthält, und einer Nachlauffraktion, die Di- und Triethylbenzol enthält, unterzogen wird.

7. Verfahren nach Anspruch 6, dadurch gekennzeichnet, daß die Kopffraktion, die Monoethylbenzol enthält, in Schritt a) zurückgeführt wird, um die Alkylierung dieses Monoethylbenzols zu Di- und Triethylbenzol zu erreichen.

8. Verfahren nach Anspruch 3 und einem der Ansprüche 4 bis 7, dadurch gekennzeichnet, daß in Schritt b) durch eine weitere Destillation-Rektifikation eine Benzinfraktion gewonnen wird, die aus gesättigten Kohlenwasserstoffen besteht und im wesentlichen kein Benzol enthält.

## Claims

1. Process for the manufacture of ethylbenzene from pure benzene or dilute benzene and a composition (B) containing ethylene, characterised in that it consists in:
a) reacting the pure benzene or dilute benzene with the composition (B) in an alkylation reaction of benzene with ethylene in which the benzene/ethylene molar ratio is between 0.3 and 1, so as to obtain an alkylate containing practically no residual benzene and containing mono-, di- and triethylbenzene;
b) subjecting this alkylate to at least one distillation/rectification in order to obtain a distillate (18) containing di- and triethylbenzene;
c) transalkylating this distillate (18) with benzene (22) in order to obtain a transalkylate (25) enriched in monoethylbenzene formed from the di- and triethylbenzene of the said distillate;
d) subjecting this transalkylate (25) to at least one distillation/rectification in order to obtain ethylbenzene (29).

2. Process according to Claim 1, characterised in that the dilute benzene contains saturated hydrocarbons.

3. Process according to Claim 1, characterised in that the dilute benzene is a light reformate.

4. Process according to one of Claims 1 to 3, characterised in that the composition (B) also contains saturated hydrocarbons, with the exclusion of unsaturated hydrocarbons other than ethylene.

5. Process according to Claim 4, characterised in that the composition (B) is a distillation fraction of a fluid catalytic cracking gas containing hydrocarbons having a number of carbons less than or equal to 2.

6. Process according to one of Claims 1 to 5, characterised in that the distillate of stage b) also contains monoethylbenzene and in that this distillate is subjected to at least one distillation/rectification in order to obtain a head fraction containing monoethylbenzene and a bottom fraction containing di- and triethylbenzene.

7. Process according to Claim 6, characterised in that the said head fraction containing monoethylbenzene is recycled to stage a) in order to obtain alkylation of this monoethylbenzene to di- and triethylbenzene.

8. Process according to Claim 3 and one of Claims 4 to 7, characterised in that a petrol fraction formed from saturated hydrocarbons, and essentially benzene-free, is recovered in stage b) by another distillation/rectification.
